# EUROPÄISCHE PATENTSCHRIFT

(11) **EP 1 663 100 B1**
(45) Veröffentlichungstag und Bekanntmachung des Hinweises auf die Patenterteilung: **18.01.2012**
(21) Anmeldenummer: 04765054.4
(22) Anmeldetag: 10.09.2004
(51) Int. Cl.: A61J 1/00, B29C 49/08, B65D 1/02, B65D 1/32

(54) **BEHÄLTER FÜR INFUSIONSFLÜSSIGKEITEN**
CONTAINER FOR INFUSION LIQUIDS
RECIPIENT POUR LIQUIDES DE PERFUSION

(30) Priorität: 16.09.2003 DE 10342742
(43) Veröffentlichungstag der Anmeldung: 07.06.2006
(73) Patentinhaber: B. Braun Melsungen AG, 34212 Melsungen (DE)
(72) Erfinder: BEINE, Joachim, 34302 Guxhagen (DE); DÖNHOFF, Torsten, 34286 Spangenberg (DE); HARMS, Volker, 34132 Kassel (DE); MAIER, Hans-Otto, 34212 Melsungen (DE)
(74) Vertreter: von Kreisler Selting Werner
(86) Internationale Anmeldenummer: PCT/EP2004/010125
(87) Internationale Veröffentlichungsnummer: WO 2005/032450

(56) Entgegenhaltungen:
- DE-A- 2 719 614
- GB-A- 2 139 551
- US-A- 3 926 341
- US-A- 5 255 808

## Beschreibung

Die Erfindung betrifft einen geformten standfähigen Behälter für Infusionsflüssigkeiten, mit Wänden, die einen Standboden und einen in einen Halsbereich übergehenden Schulterbereich bilden.

Bekannt sind Glasbehälter, die Infusionsflüssigkeit enthalten, welche einem Patienten über ein Schlauchsystem zugeführt wird. Derartige Behälter sind mit einem gummielastischen Stopfen verschlossen, der von dem Dorn eines Übertragungsgerätes durchstochen werden kann. Der Behälter wird dann mit nach unten gerichteter Öffnung aufgehängt, so dass die Flüssigkeit durch das Entnahmegerät in kontrollierter Weise auslaufen kann. Hierbei wird durch einen Belüftungskanal des Einstechdornes Luft in den Behälter hineingelassen, um das von der Flüssigkeit freigemachte Volumen aufzufüllen und einen Unterdruck im Behälter zu vermeiden.

Derartige starre Behälter eignen sich nicht für Druckinfusionen, bei denen die Infusionsflüssigkeit durch Anwendung von äußerem Druck aus dem Behälter herausgedrückt wird. Druckinfusionen werden verabreicht bei Patienten im hypovolämischen Zustand nach Verlust größerer Mengen Blut, beilspielsweise nach einem Verkehrsunfall, um dem Patienten in kurzer Zeit eine große Flüssigkeitsmenge zuzuführen. Für solche Druckinfusionen eigenen sich Folienbeutel, in denen die Infusionsflüssigkeit enthalten ist. Die Folienbeutel enthalten keine Luft, und ihr Volumen passt sich an das jeweilige Flüssigkeitsvolumen an. Allerdings ist die Herstellung geeigneter Folienbeutel sehr aufwendig. Als Material werden mehrschichtige Verbundfolien eingesetzt, wobei mindestens eine der Lagen besondere Barriereeigenschaften aufweist.

In der Herstellung günstiger sind geformte Behälter, die im Blow-Fill-Seal-Verfahren (BFS) hergestellt und abgefüllt werden, wobei alle Arbeitsschritte - das Blasformen des Behälters, seine Füllung und das hermetische Verschließen - im formgebenden Werkzeug stattfinden.

Bekannt ist ferner ein unter der Bezeichnung Ecoflac plus^{®} von der Firma B. Braun Melsungen AG vertriebener Behälter von ovalem Grundriss, der im BFS-Verfahren hergestellt wird und dessen Wand sich bei Anwendung einer Infusion ohne zusätzliche Belüftung einschnürt. Allerdings bleiben der Standboden und der Schulterbereich dieses Behälters aufgrund ihrer durch die Formgebung hervorgerufenen Stabilität undeformiert, so dass nur der Mittelbereich eingeschnürt wird. Dies bedeutet, dass im Standboden und im Schulterbereich selbst bei kollabiertem Behälter Hohlräume bestehen bleiben. Diese Hohlräume müssen mit Luft gefüllt sein, um mit dem Behälter eine normale Schwerkraftinfusion durchführen zu können. Eine solche Schwerkraftinfusion erfordert, dass im Behälter stets ein Luftvolumen vorhanden ist. Das benötigte Luftvolumen ist wegen der Formstabilität der genannten Endbereiche des Behälters relativ groß. Andererseits muss verhindert werden, dass Luft in das Schlauchsystem des Übertragungsgerätes eindringt, weil dann für den Patienten die Gefahr einer Luftembolie besteht.

In JP 2002 282 335 A ist ein Infusionsbehälter beschrieben, der dem Oberbegriff des Patentanspruchs 1 entspricht. Der Infusionsbehälter besteht aus einem Formkörper, der im leeren Zustand vor dem Befüllen und nach dem Entnehmen der Flüssigkeit zusammengefaltet werden kann. Während des Einfüllens der Infusionslösung und beim Transport nach dem Befüllen, kann der Behälter jedoch aufrecht stehen. Der Behälter weist an zwei gegenüberliegenden Seitenflächen umgekehrt - y-förmige Außenfaltlinien auf sowie eine die Außenfaltlinie verbindende querlaufende Innenfaltlinie. Beim Hochfalten des Behälterbodens weichen die Außenfaltlinien nach außen auf, während die Innenfaltlinie einknickt. Im Halsbereich des Behälters findet keine Faltung statt. Der Behälter wird durch manuelle Einwirkung deformiert, wobei das Verformungsverhalten durch den Ort der Druckausübung beeinflusst wird.

In DE 37 27 972 A1 ist ein als Beutel ausgebildeter Flüssigkeitsbehälter zur Aufnahme von Getränken, wie Orangensaft und Erfrischungsgetränke, beschrieben. Der Beutel ist ein Seitenfaltenbeutel mit mehreren vertikalen Schweißnähten. Der Boden besteht aus übereinander gefalteten Laschen. Der Beutel ist nur im gefüllten Zustand selbststehend. Zum Aufhängen ist er nicht vorgesehen.

DE 699 03 510 T2 beschreibt einen generell würfelförmigen Großbeutel zur Verwendung in der biopharmazeutischen Industrie, der als Faltenbalgbeutel aus verschweißten Folien hergestellt ist. Es handelt sich nicht um einen geformten Behälter. Der Behälter weist auch keinen Halsbereich und keinen Schulterbereich auf.

Ein Seitenfaltenbeutel für medizinische Zwecke ist in DE 699 00 761 T2 beschrieben. Der Seitenfaltenbeutel hat im gefüllten Zustand quaderförmige Gestalt, wobei der Boden aus dreieckförmigen Laschen besteht, die durch Siegelnähte verbunden sind. Folienbeutel mit zahlreichen Siegelnähten erfordern eine relativ aufwendige Herstellung.

In DE 43 15 966 ist ein zusammenlegbarer Behälter beschrieben, der aus einem einstückigen Formteil besteht und längslaufende Seitenfalten aufweist. Der Behälter ist dazu bestimmt, manuell zusammengedrückt zu werden, um bei der Abfallentsorgung ein geringes Volumen zu beanspruchen.

Ein geformter standfähiger Behälter für Infusionsflüssigkeiten, der im wesentlichen dem Oberbegriff des Patentanspruchs 1 entspricht, ist beschrieben in U.S. 3,926,341 A. Dieser Behälter ist im Extrusions-Blasformverfahren hergestellt. Er besteht aus einer halbstarren Kunststoffflasche, die mit Biegelinien versehen ist. Längslaufende Biegelinien erzeugen jeweils zwischen zwei Außenfalten eine Innenfalte, so dass die ebenen Seitenwände sich beim Leeren des Behälters aneinander annähern, während die Schmalwände sich ziehharmonikaartig zusammenlegen. Die Flasche hat einen Standboden, der beim Entleeren zum Flascheninneren eingezogen wird. Ferner hat die Flasche einen Schulterbereich, der in einen Halsbereich übergeht und frei von Biegelinien ist. Der Schulterbereich ist daher steif.

Der Erfindung liegt die Aufgabe zugrunde, einen geformten Behälter zu schaffen, der einfach und kostengünstig herstellbar ist und Druckinfusionen gestattet, ohne ein größeres Luftvolumen zu erfordern.

Der geformte standfähige Behälter nach der vorliegenden Erfindung weist die Merkmale des Anspruchs 1 auf.

Unter einem "geformten Behälter" wird ein Behälter verstanden, dessen Formgebung bei der Herstellung bestimmt wird und der deswegen eine vorbestimmte Form hat und diese beim Auflegen auf eine Unterlage auch einhält, im Gegensatz zu einem Folienbeutel. Diese Standfähigkeit kann durch angeformte Noppen positiv beeinflusst werden. Jedoch ist der erfindungsgemäße Behälter im Falle einer Volumenentnahme aus dem Behälterinneren durch den äußeren Luftdruck (Umgebungsdruck) deformierbar. Ferner ist der Behälter durch Ausüben von Kraft auf die Behälterwandung deformierbar, um eine Druckinfusion durchzuführen.

Dadurch, dass sich bei dem geformten Behälter bei einer Flüssigkeitsentnahme nicht nur der Mittelbereich verformt, sondern auch die aus dem Standboden und dem Schulterbereich bestehenden Endbereiche, wird das benötigte Luftvolumen zum Leerlaufen des Behälters in erheblichem Maße reduziert. Es ist daher erforderlich, in dem Behälter nur ein minimales Luftvolumen bereitzustellen, das diesem Restvolumen entspricht. Der Behälter ist im Füllzustand nahezu luftfrei. Dies bedeutet, dass das Luftvolumen maximal 15% des Behältervolumens ausmacht. Bei den bekannten geformten Behältern beträgt das Luftvolumen je nach Füllvolumen/Behältergröße zwischen 20 und 40% des Behältervolumens. Da der erfindungsgemäße Behälter im befüllten Zustand noch teilweise eingedrückt ist und somit noch eine Volumenreserve für die Zugabe einer Zuspritzmenge hat, ist ein größeres Luftvolumen nicht erforderlich.

Bei einer unbelüfteten Entnahme des Inhalts läuft der geformte Behälter trotz fehlendem Luftvolumen gleichmäßig leer. Daher weist er eine gute Bilanzierbarkeit auf.

Der erfindungsgemäße Behälter kann relativ leicht hergestellt werden, beispielsweise im BFS-Verfahren, und zwar entweder einschichtig oder mehrschichtig. Die Vorteile eines Folienbeutels, nämlich minimales Rest- bzw. Luftvolumen, und eines im BFS-Verfahren hergestellten Behälters, nämlich kostengünstige Fertigung, werden in dem erfindungsgemäßen Behälter vereinigt.

Gemäß einer bevorzugten Ausgestaltung der Erfindung sind die Biegelinien durch eine gezielte Manipulation im Wanddickenverlauf in den Wänden gebildet. Eine solche Biegelinie kann im Blasverfahren geformt werden. Hierzu muss die Blasform entsprechende Stege aufweisen, die eine Abbildung der genannten Faltlinien bewirken.

Die Faltbarkeit des Schulterbereichs wird vorzugsweise dadurch erreicht, dass mehrere Biegelinien in einer Gruppe derart angeordnet sind, dass sie eine ziehharmonikaartige Faltung ergeben. Die Formstabilität des Schulterbereichs ist normalerweise groß, weil der Schulterbereich, je nach Behältergeometrie, pyramiden- oder kegelstumpfförmig ist. Die Formstabilität wird noch dadurch erhöht, dass der Schulterbereich durch Körperkanten mit erhöhter Biegesteifigkeit begrenzt wird. Das relativ große Volumen, das der Schulterbereich einschließt, wird durch die ziehharmonikaartige Faltung und das entsprechende Flachlegen erheblich reduziert.

Im Standboden ist eine Biegelinie als Querfalte ausgebildet, die beim Flachlegen nach außen wandert. Generell ist der Standboden so gestaltet, dass er sich beim Flachlegen nach außen (und nicht nach innen) verlagert, weil dadurch das Behältervolumen am stärksten reduziert werden kann.

Die Erfindung betrifft ferner ein Verfahren zum Füllen eines Behälters mit Infusionsflüssigkeit nach seiner Formung. Wenn in einen Infusionsbehälter eine Flüssigkeit, z. B. ein Medikament, zugespritzt wird, muss der geschlossene Behälter ein zusätzliches Flüssigkeitsvolumen aufnehmen. Wenn es sich um einen starren Behälter handelt, erhöht sich dadurch der Innendruck des Behälters. Handelt es sich um einen flexiblen Beutel, so wird die Beutelwand zusätzlich gedehnt.

Dem neuen Verfahren liegt die Aufgabe zugrunde, ein Verfahren zum Füllen eines Behälters anzugeben, mit welchem sichergestellt werden kann, dass im Falle des Zuspritzens einer Flüssigkeit keine wesentliche mechanische Beanspruchung des Behälters erfolgt.

Dies wird nach dem Anspruch 9 dadurch erreicht, dass der Behälter nach dem Formen zur Reduzierung seines Volumens eingedrückt und in diesem Zustand befüllt wird, wobei durch das Eindrücken eine Volumenreserve für die Aufnahme einer später zugeführten Zuspritzmenge bereitgestellt wird. Das Eindrücken des Behälters bedeutet, dass dieser beim Befüllvorgang nicht sein gesamtes Volumen zur Verfügung stellt. Vielmehr wird ein Teil einer Wand zwangsweise so deformiert, dass die Wand den im wesentlichen spannungsfreien Endzustand nicht einnimmt, sondern nach innen gewölbt ist. Der Behälter wird also nur unvollständig befüllt, um anschließend noch eine Zuspritzmenge aufnehmen zu können, wodurch er sein vorgesehenes Endvolumen einnimmt.

Im folgenden wird unter Bezugnahme auf die Zeichnungen ein Ausführungsbeispiel der Erfindung näher erläutert. Die nachfolgende Beschreibung ist nicht so zu verstehen, dass dadurch der Schutzbereich des Patents eingeschränkt würde. Dieser wird vielmehr durch die Ansprüche bestimmt.

Es zeigen:
- Fig. 1: eine perspektivische Darstellung eines formstabilen Behälters nach der Erfindung,
- Fig. 2: einen Querschnitt durch eine Biegelinie entlang der Linie II-II von Figur 1,
- Fig. 3: eine perspektivische Darstellung des Behälters nach Entnahme des Inhalts,
- Fig. 4: einen aufgeschnittenen mehrschichtigen Behälter nach der Erfindung zur Verdeutlichung des Schichtaufbaus,
- Fig. 5: ein Vorformling zur Herstellung eines mehrschichtigen Behälters durch (Co-)Extrusionsblasformen, wobei der Vorformling kreisrunden Querschnitt hat, und
- Fig. 6: eine Ausführungsform eines Vorformlings für einen mehrschichtigen Behälter, wobei der Vorformling einen länglichen Querschnitt hat mit parallelen Seitenwänden und gerundeten Endwänden.

Der dargestellte Behälter 10 weist einen langgestreckten hohlen Körper 11 auf, der durch vier Wände 12,13 begrenzt ist. Der Körper 12 hat eine rechteckige Form. Er geht an seinem einen Ende in einen pyramidenstumpfförmigen Schulterbereich 14 über, an den sich ein parallel zu den Wänden 12 verlaufender langgestreckter rechteckförmiger Halsbereich 15 anschließt. Auf dem Halsbereich 15 sitzt ein Portsystem 16 mit zwei Ports 16a,16b, von denen jeder eine durchstechbare Membran enthaltende Entnahmeöffnung aufnimmt. Jeder Port weist ein Abreißteil 17a,17b auf, welches abgerissen werden kann, um die Membran freizulegen, damit der Einstechdorn eines Übertragungsgerätes hineingestochen werden kann. Einer der Ports dient als Entnahmeport und der andere als Zuspritzport. Seitlich an den Ports sind abstehende Laschen vorgesehen, wodurch der Anschluss von Monovial-Behältern ermöglicht wird.

Der Standboden 20 des Körpers 12 enthält eine Bodenwand 21. Von dieser steht eine Aufhängelasche 22 ab, um den Behälter mit nach unten gerichteter Entnahmeöffnung aufhängen zu können. Der Standboden kann zusätzlich zum besseren Handling des Behälters noch Standnoppen 24 beinhalten. Die Standnoppen 24 schaffen einen Freiraum zur Unterbringung der seitlich abgebogenen Aufhängelasche und bewirken einen sicheren und kippfreien Stand des Behälters.

Der Behälter 10 ist, mit Ausnahme des Portsystems 16, einstückig hergestellt und mit Infusionsflüssigkeit befüllt. Üblicherweise besteht er aus Materialien polyolefinischer Natur, z.B. LDPE, LLDPE, PP.

Um die für eine Druckinfusion erforderliche Deformierbarkeit zu bewirken, ist der Behälter mit Biegelinien BL versehen, die sich vornehmlich im Standboden 20 und im Schulterbereich 14 erstrecken. Eine Biegelinie 25a läuft quer über den Standboden 20. Die Biegelinie 25a weist einen Mittelabschnitt auf, von dem die Lasche 22 absteht. Von den beiden Enden des Mittelabschnitts gehen weitere Biegelinien 30 ab, die sich strahlenförmig bis in die Ecken der Bodenwand 21 erstrecken. Eine Gruppe von Biegelinien 26, die nach Art eines Rechens angeordnet sind, erstreckt sich im Schulterbereich 14 und von dort bis in den Körper 12 hinein. Die Biegelinien 26 verlaufen generell in Längsrichtung des Behälters, und sie sind so angeordnet, dass sie eine ziehharmonikaartige Faltung ergeben, wobei die Übergangskante 27 zwischen Körper 12 und Schulterbereich 14 gebrochen wird.

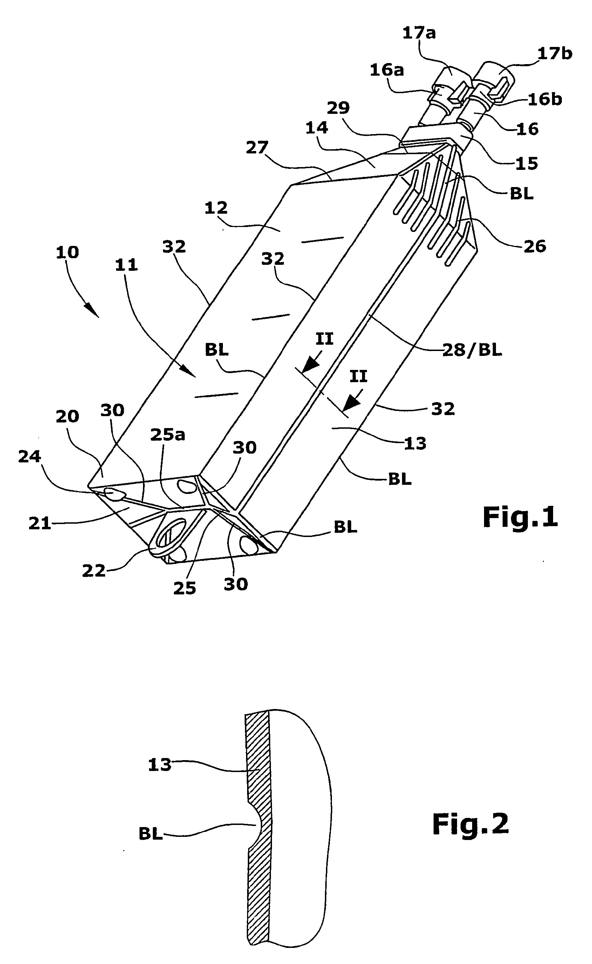

Eine oder auch mehrere weitere Biegelinien 28 verlaufen entlang einer Wand 13 in Längsrichtung des Behälters. An den längslaufenden Kanten des Behälters sind weitere Biegelinien 32 vorgesehen. Während die Biegelinie 28 eine Innenfalte bildet, bilden die Biegelinien 32 Außenfalten, welche die Ecken des Körpers 11 verbinden. Eine oder auch mehrere entsprechende Biegelinien verlaufen auf der in Fig. 1 nicht sichtbaren gegenüberliegenden Wand. Beim Flachlegen des Behälters wandern die Biegelinien 28 nach innen und die im Boden befindliche Biegelinie 25 nach außen.

Figur 2 zeigt einen Querschnitt der Wand 13 mit der darin ausgebildeten Biegelinie BL. Entlang der Biegelinie ist die Wandstärke reduziert, so dass dort die Biegesteifigkeit erheblich herabgesetzt ist. Wird der Behälter im Blasverfahren hergestellt, so kann die Biegelinie BL dadurch erzeugt werden, dass in der Wand der Blasform eine dem Formhohlraum zugewandte Rippe vorgesehen ist. Dadurch entsteht die Biegelinie an der Behälteraußenseite, während die Behälterinnenseite glatt ist. Prinzipiell kann die Falte aber an der Behälterinnenseite durch werkzeugseitige Vertiefungen erzeugt werden.

Der beschriebene Behälter wird vollständig oder nahezu vollständig mit Flüssigkeit gefüllt. Bei dem beschriebenen Ausführungsbeispiel befindet sich der Flüssigkeitsspiegel 29 bei aufrecht stehendem Behälter im oberen Teil des Schulterbereichs 14. Darüber befindet sich nur eine relativ geringe Luftmenge, die ausreicht, um das bei deformiertem Behälter noch vorhandene Behältervolumen auszufüllen.

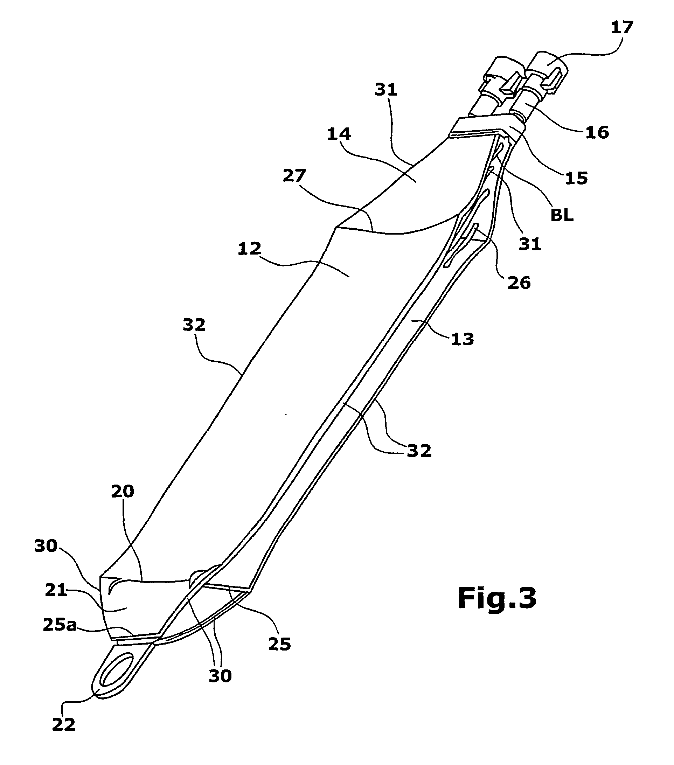

Figur 3 zeigt den Behälter im kollabierten Zustand nach Entnahme des Inhalts. Man erkennt, dass die Seitenwände 13 wie die Wände eines Seitenfaltenbeutels längs gefaltet und mit ihren Hälften gegeneinander gelegt sind, wobei die Biegelinie 28 eine Innenfalte und die Biegelinien 32 Außenfalten bilden. Diese Seitenfaltung setzt sich in den Standboden 20 und den Schulterbereich 14 hinein fort. Auch im Schulterbereich 14 befinden sich Biegelinien, die eine Fortsetzung der längslaufenden Biegelinien 28 und 32 des Körpers 11 bilden.

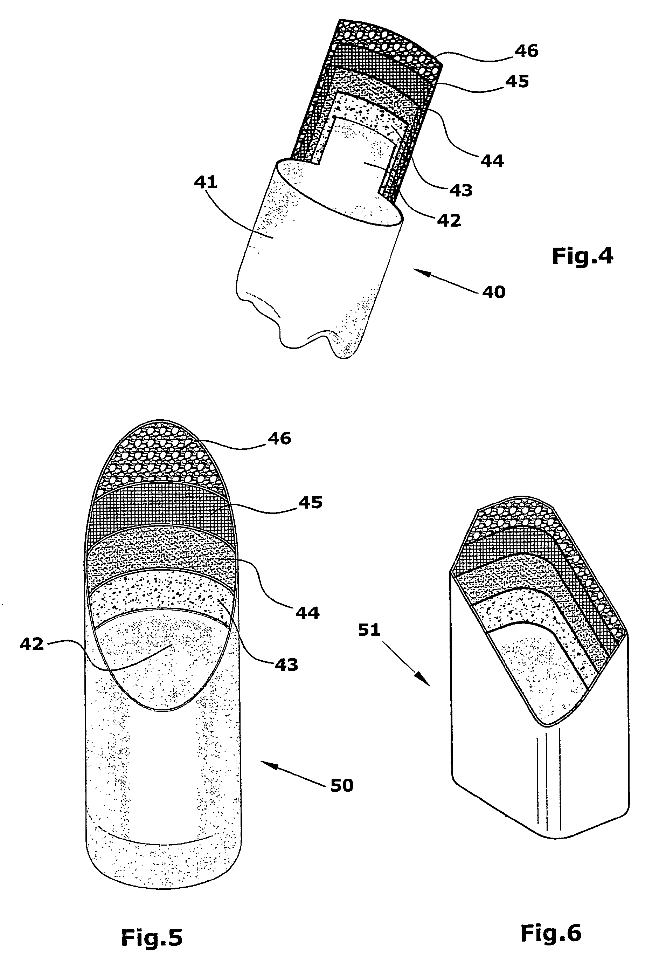

In Figur 4 ist ein Behälter 40 im aufgeschnittenen Zustand dargestellt, der eine zylindrische Behälterwand 41 aufweist, welche aus einer oder mehreren Schichten bestehen kann. Die Zahl der Schichten beträgt zwischen 1 und 10. Bei dem dargestellten Ausführungsbeispiel sind die folgenden Schichten vorhanden:
- eine Innenschicht 42 aus Polypropylen (PP), vorzugsweise Polypropylen-Copolymer (CoPP),
- eine Haftvermittlerschicht 43 aus modifiziertem Polyolefin mit funktionellen Anhydrid-Gruppen,
- mindestens eine Barriereschicht 44 aus Polyamid (PA), vorzugsweise Polyamid-Copolymer (CoPA) und/oder Ethylen/Vinylalkohol-Copolymer (EVOH),
- eine äußere Haftvermittlerschicht 45, die ähnlich aufgebaut ist wie die innere Haftvermittlerschicht 43,
- eine Außenschicht 46 aus Polyamid (PA), vorzugsweise Polyamid-Copolymer (CoPA) und/oder Polyester (PET), vorzugsweise CoPolyester (CoPET).

Die Gesamtwandstärke der Behälterwand 41 beträgt von 0,1 mm bis 0,7 mm. Etwa 40% bis 70% der gesamten Wandstärke entfallen auf die Innenschicht 42 und 10% auf die Barriereschicht 44 und die Haftvermittlerschichten 43 und 45. Der Rest der Wandstärke entfällt auf die Außenschicht 46.

Zur Herstellung des Behälters nach Figur 4 wird zunächst der in Figur 5 dargestellte mehrschichtige Vorformling im (Co-)Extrusionsverfahren hergestellt. Der Vorformling 50 besteht aus einem geraden Rohr, das hier kreisrunden Querschnitt hat und die einzelnen Schichten 42 bis 46 in koaxialer rohrförmiger Anordnung enthält. Der Vorformling wird in einer Blasform aufgeweitet und in seine endgültige Form gebracht. Dabei wird die Wandstärke auf 0,1 mm bis 0,7 mm reduziert. Die eingesetzten Materialien sind so gewählt, dass der Behälter in Kombination mit der geringen Wandstärke eine hohe Transparenz aufweist, die deutlich über der Transparenz üblicher (co-)extrusionsblasgeformter Behälter für Infusionslösungen mit polyolefinischem Aufbau (PP/PE/COC) abweicht. Zusätzlich zu den Materialeigenschaften kann eine signifikante Steigerung der Transparenz durch eine axiale Reckung des Vorformlings vor dem Blasvorgang erzielt werden.

Figur 6 zeigt einen anderen Vorformling 50, der einen vom runden Querschnitt abweichenden profilierten Querschnitt aufweist. Der Querschnitt des Vorformlings 51, der die gleichen Schichten aufweist wie der Vorformling 50, ist hier derjenige eines langgestreckten Rechtecks mit stark abgerundeten Ecken. Die (nicht dargestellte) Extrusionsdüse im Werkzeugkopf des Extrusionswerkzeugs hat eine entsprechende Profilform. Die Materialverteilung im Vorformling 51 ist so gewählt, dass bei dem anschließenden Aufweiten Blasformprozess eine umfangsmäßig gleichmäßige Wandstärke des Behälters entsteht.

Die Formung des in Figur 4 dargestellten Behälters erfolgt vorzugsweise so, dass dieser Behälter in den Wänden des Standbodens und/oder des Schulterbereichs Biegelinien aufweist. Bei der erwähnten geringen Wandstärke besteht auch die Möglichkeit, dass der Behälter sich im Bereich des Standbodens und im Schulterbereich bei Flüssigkeitsentnahme aus dem Behälter verformt, ohne dass derartige Biegelinien vorhanden sind. Vorgeformte Biegelinien bzw. Schwächungsbereiche erleichtern jedoch das definierte Kollabieren und eine ordnungsgemäße Faltung des Behälters.

## Patentansprüche

1. Geformter standfähiger Behälter für Infusionsflüssigkeiten, mit Wänden (12,13), die einen das Aufstellen des Behälters ermöglichenden flachen Standboden (20) und einen in einen Halsbereich (15) übergehenden Schulterbereich (14) bilden, wobei in den Wänden des Standbodens (20) und/oder des Schulterbereichs (14) Biegelinien (BL) geformt sind, welche derart angeordnet und ausgebildet sind, dass sie bei unbelüfteter Flüssigkeitsentnahme aus dem Behälter (10) ein Flachlegen des die Biegelinien aufweisenden Bereichs bewirken,
**dadurch gekennzeichnet,**
**dass** im Standboden (20) eine quer über den Standboden (20) verlaufende Biegelinie (25a) als Querfalte ausgebildet ist, die beim Flachlegen nach außen wandert.

2. Behälter nach Anspruch 1, **dadurch gekennzeichnet, dass** die Biegelinien (BL) durch Schwächungsbereiche in den Wänden (13) gebildet sind.

3. Behälter nach Anspruch 1 oder 2, **dadurch gekennzeichnet, dass** mehrere Biegelinien (BL) in einer Gruppe von Biegelinien (26) derart angeordnet sind, dass sie eine ziehharmonikaartige Faltung ergeben.

4. Behälter nach einem der Ansprüche 1 - 3, **dadurch gekennzeichnet, dass** mehrere Biegelinien (28, 32) sich als Längsfalten vom Standboden (20) bis in den Schulterbereich (14) erstrecken.

5. Behälter nach einem der Ansprüche 1 - 4, **dadurch gekennzeichnet, dass** der gefüllte Behälter ein Luftvolumen von maximal 15% des Behältervolumens aufweist.

6. Behälter nach einem der Ansprüche 1 - 5, **dadurch gekennzeichnet, dass** das Füllvolumen des Behälters 1 ml bis 5000 ml beträgt.

7. Behälter nach einem der Ansprüche 1 - 6, **dadurch gekennzeichnet, dass** der Halsbereich (15) mit einem Portsystem (16) versehen ist, das eine durchstechbare Membran aufweist.

8. Behälter nach einem der Ansprüche 1 - 7, **dadurch gekennzeichnet, dass** der Standboden (20) eine abstehende Haltelasche (22) aufweist.

9. Verfahren zum Füllen eines Behälters nach einem der Ansprüche 1 - 8 mit Infusionsflüssigkeit, **dadurch gekennzeichnet, dass** der Behälter nach dem Formen zur Reduzierung seines Volumens eingedrückt und in diesem Zustand befüllt und verschlossen wird, wobei durch das Eindrücken eine Volumenreserve für die Aufnahme einer später zugeführten Zuspritzmenge bereitgestellt wird.

## Claims

1. A molded container capable of standing upright, for infusion liquids, said container comprising walls (12, 13) which form a flat standing bottom (20) allowing the container to stand upright and a shoulder portion (14) passing into a neck portion (15), fold lines (BL) being formed in the walls of the standing bottom (20) and/or the shoulder portion (14), the fold lines being arranged and configured such that they cause a flattening of the portion having the fold lines when liquid is withdrawn from the container (10) without ventilation,
**characterized in that**
the standing bottom (20) is formed with a fold line (25a) as a transverse fold that moves outward during flattening.

2. The container of claim 1, **characterized in that** the fold lines (BL) are formed by weakened portions in the walls (13).

3. The container of claim 1 or 2, **characterized in that** a plurality of fold lines (BL) is arranged in a group of fold lines (26) such that they form an accordion-like folding.

4. The container of one of claims 1 - 3, **characterized in that** a plurality of fold lines (28, 32) extends as longitudinal folds from the standing bottom (20) into the shoulder portion (14).

5. The container of one of claims 1 - 4, **characterized in that** the filled container contains a volume of air of at most 15% of the container volume.

6. The container of one of claims 1 - 5, **characterized in that** the filling volume of the container is 1 ml to 5000 ml.

7. The container of one of claims 1 - 6, **characterized in that** the neck portion (15) is provided with a port system (16) comprising a pierceable membrane.

8. The container of one of claims 1 - 7, **characterized in that** the standing bottom (20) comprises a projecting suspension lug (22).

9. A method for manufacturing a molded standing container for infusion liquids of one of claims 1-8, **characterized in that** the container is impressed after molding to reduce its volume and is filled and closed in this state, the impressing providing for a backup volume for receiving a supplementary volume injected later.

## Revendications

1. Récipient moulé pour liquides de perfusion, apte à rester debout, comprenant des parois (12, 13) formant un fond de support (20) plat permettant de dresser ledit récipient, et formant une partie d'épaulement (14) passant dans une partie de cou (15), les parois du fond de support (20) et/ou de la partie d'épaulement (14) étant formées avec des lignes de pli (BL) qui sont disposées et formées de sorte qu'elles causent la mise à plat de la partie comprenant les lignes de pli quand l'on enlève du liquide du récipient (10) sans aération,
**caractérisé en ce que**
une ligne de pli (25a), s'étendant transversalement sur ledit fond de support (20), est formée dans ledit fond de support comme pli transversale qui se déplace vers l'extérieur pendant la mise à plat.

2. Récipient selon la revendication 1, **caractérisé en ce que** lesdites lignes de pli (BL) sont formées par des lignes d'affaiblissement dans les parois (13).

3. Récipient selon les revendications 1 ou 2, **caractérisé en ce que** plusieurs lignes de pli (BL) sont disposées dans un groupe de lignes de pli (26) de sorte qu'elles forment un pliage du type accordéon.

4. Récipient selon les revendications 1-3, **caractérisé en ce que** plusieurs lignes de pli (28, 32) s'étendent, comme plis longitudinales, du fond de support (20) jusque dans ladite partie d'épaulement (14).

5. Récipient selon les revendications 1 - 4, **caractérisé en ce que** le récipient rempli a un volume d'air qui est au maximum 15% du volume du récipient.

6. Récipient selon les revendications 1 - 5, **caractérisé en ce que** le volume de remplissage du récipient est entre 1 ml et 5000 ml.

7. Récipient selon les revendications 1 - 6, **caractérisé en ce que** la partie de cou (15) est munie d'un système de ports (16) comprenant une membrane perçable.

8. Récipient selon les revendications 1 - 7, **caractérisé en ce que** le fond de support (20) comprend une patte de suspension (22) en saillie.

9. Procédé de remplissage d'un récipient selon les revendications 1-8 avec liquide de perfusion, **caractérisé en ce qu'**après moulage, ledit récipient est pressé pour réduire son volume et qu'il est rempli et fermé dans cet état, le fait qu'il est pressé fournissant une réserve de volume pour recevoir une quantité injectée plus tard.
